# EUROPEAN PATENT SPECIFICATION

(11) **EP 1 461 449 B1**
(45) Date of publication and mention of the grant of the patent: **02.11.2011**
(21) Application number: 02806289.1
(22) Date of filing: 31.12.2002
(51) Int. Cl.: C12Q 1/00, G01N 33/48, G01N 33/53, G01N 33/00, G01N 21/00, G01N 21/75, G01N 21/76, G01N 33/574, G01N 33/554, G01N 33/569, A61K 49/00

(54) **SYSTEM FOR MONITORING BACTERIAL TUMOR TREATMENT**
SYSTEM ZUR ÜBERWACHUNG EINER BAKTERIELLEN TUMORBEHANDLUNG
SYSTEME DE SURVEILLANCE D'UN TRAITEMENT BACTERIEN D'UNE TUMEUR

(30) Priority: 31.12.2001 US 345699 P
(43) Date of publication of application: 29.09.2004
(73) Proprietor: AntiCancer, Inc., San Diego, CA 92111 (US)
(72) Inventor: ZHAO, Ming, M.D., Ph.D., San Diego, CA 92121 (US); LI, Xiao-Ming, M.D., Ph.D., San Diego, CA 92117 (US); YANG, Meng, M.D., Ph.D., San Diego, CA 92129 (US); XU, Mingxu, M.D., La Jolla, CA 92037 (US); JIANG, Ping, M.D., San Diego, CA 92122 (US); LI, Lingna, M.D., San Diego, CA 92130 (US)
(74) Representative: Fisher, Adrian John
(86) International application number: PCT/US2002/041822
(87) International publication number: WO 2003/057007

(56) References cited:
- WO-A-01/25399
- WO-A-02/060941
- WO-A1-03/006069
- ZHENG L ET AL: "TUMOR AMPLIFIED PROTEIN EXPRESSION THERAPY: SALMONELLA AS A TUMOR-SELECTIVE PROTEIN DELIVERY VECTOR" ONCOLOGY RESEARCH, PERGAMON PRESS, NEW YORK, NY, US, vol. 12, no. 3, 2000, pages 127-135, XP009020983 ISSN: 0965-0407
- YANG M ET AL: "Whole-body optical imaging of green fluorescent protein-expressing tumors and metastases." PROCEEDINGS OF THE NATIONAL ACADEMY OF SCIENCES OF THE UNITED STATES OF AMERICA. 1 FEB 2000, vol. 97, no. 3, 1 February 2000 (2000-02-01), pages 1206-1211, XP000904536 ISSN: 0027-8424
- YANG M ET AL: "Whole-body and intravital optical imaging of angiogenesis in orthotopically implanted tumors" PROCEEDINGS OF THE NATIONAL ACADEMY OF SCIENCES OF THE UNITED STATES OF AMERICA 27 FEB 2001 UNITED STATES, vol. 98, no. 5, 27 February 2001 (2001-02-27), pages 2616-2621, XP002370675 ISSN: 0027-8424
- MIKI KENJI ET AL: "Methioninase cancer gene therapy with selenomethionine as suicide prodrug substrate" CANCER RESEARCH, vol. 61, no. 18, 15 September 2001 (2001-09-15), pages 6805-6810, XP002370676 ISSN: 0008-5472
- ZHAO, MING [REPRINT AUTHOR] ET AL: "GFP - and RFP -labeled bacteria to target tumors." PROCEEDINGS OF THE AMERICAN ASSOCIATION FOR CANCER RESEARCH ANNUAL MEETING, (MARCH, 2002) VOL. 43, PP. 586. PRINT. MEETING INFO.: 93RD ANNUAL MEETING OF THE AMERICAN ASSOCIATION FOR CANCER RESEARCH. SAN FRANCISCO, CALIFORNIA, USA. APRIL 06-10, 2002., 2002, XP002370678

## Description

### Technical Field

The invention relates to the use of bacteria as vectors for effecting tumor treatment and methods to monitor localization and efficacy. In more detail, the invention concerns the use of fluorescent proteins delivered in bacteria to monitor the delivery and efficacy of antitumor drugs.

### Background Art

The use of green fluorescent protein to visualize cancer progression and metastasis is by now well established. See, for example, Hoffman, R.M., Methods in Enzymology (1999) 302:20-31 (P. Michael Conn, ed., Academic Press, San Diego). The use of whole body imaging to chart real time progression and to assess the efficacy of proposed protocols for treating tumors is disclosed in U.S. patent 6,251,384.

Included in the advantages of green fluorescent protein are the features that it does not require any substrates or cofactors in order to fluoresce and its expression in living cells does not cause any apparent biological damage. In addition, the level of fluorescence emitted makes this a particularly sensitive technique. Whole body images are obtainable using simple equipment, e.g., 490 nm excitation from a xenon or mercury lamp along with image capture by a CCD color video camera, not to mention direct visual observation. These permit real-time investigations of tumor growth and metastasis. See, for example, Yang, M., et al., Proc. Natl. Acad. Sci. USA (2000) 97:1206-1211.

U.S. provisional application 60/304,223, filed 9 July 2001, describes the labeling of *E. coli* and other bacteria using fluorescent proteins in order to monitor infection and evaluate treatments therefor. The ability to label bacteria in the benign manner there described permits adaptation of the technique to the recently described protocols for combination bacteriolytic therapy for treatment of tumors. The technique of labeling bacteria is also described by Zhao, M., et al., Proc. Natl. Acad. Sci. USA (2001) 98:9814-9818, and Yang, M., et al., Proc. Natl. Acad. Sci. USA (2000) 97:12278-12282.

The ability to label bacteria and their products with fluorescent protein provides a dramatic improvement to a newly developed approach to the treatment of solid tumors described by Dang, L.H., et al., Proc. Natl. Acad Sci. USA (2001) 98:15155-15160. This approach to tumor treatment, called by its developers "combination bacteriolytic therapy" or "COBALT" takes advantage of the properties of certain anaerobic bacteria to grow preferentially in the anaerobic environment of the interior of solid tumors. The article surveyed the growth characteristics of a large number of strains of *Bifidobacteria, Lactobacilli,* and *Clostridia,* all of which selectively proliferate in hypoxic regions of tumors, citing a multiplicity of papers that establish this fact. The bacteria themselves could be injected intratumorally, but in order to provide a more convenient mode of administration, the authors took advantage of the fact, also established in the art, that although the live bacteria are directly toxic when injected intravenously, spores of these bacteria could be injected intravenously into normal mice without causing immediate side effects. This was not entirely true; intravenous administration of the spores was ultimately lethal unless the ability of the bacteria to secrete toxins was crippled. The authors succeeded in incapacitating this ability in *Clostridium novyi* by taking advantage of the fact that the single toxin gene is located within a phage episome, so that heat-treated bacteria showed a loss of phage. A strain was thus developed which was nontoxic when the spores were administered intravenously.

Another problem encountered was the propensity of many of the anaerobic bacterial strains to cluster into just a small number of colonies in the hypoxic tumor background. It was found that *Clostridium novyi* and *C. sordellii* were able to grow in a dispersed fashion in the hypoxic tumor areas. Thus, by creating a nontoxic version of *C. novyi* (*C, novyi-NT*) Dang, *et al.,* were able to introduce spores intravenously which home to the tumors and by virtue of their growth uniquely in the necrotic areas, were able to destroy the surrounding viable tumor cells. The ability of these bacteria to reach hypoxic, necrotic areas (which were found to account for 25%-75% of tumor volume in biopsied samples of >1 cm³) is crucial. Necrotic areas (which are necrotic due to lack of oxygen) are typically surrounded by viable cells. These cells are not reached by chemotherapeutic agents since there is no circulating blood to transport them and are relatively immune to radiation because oxygen is required for radiation to exert its lethal affect. Thus, the ability of anaerobic bacteria to thrive specifically in hypoxic tumor environments makes them a valuable delivery system for therapy. The growth of the bacteria themselves, indeed, is able to cause tumor regression as was demonstrated by Dang, *et al.* See also Yazawa, K., et al., Cancer Gene Therapy (2000) 17:269-274; Yazawa, K., et al., Breast Cancer Res. & Dev. (2001) 66:6665-170.

A similar approach was earlier described by Low, K.B., et al., Nature Biotechnology (1999) 17:37-41. In this case, *Salmonella* strains had been developed as antitumor agents as they are able to survive in anaerobic environments and preferentially proliferate in the hypoxic areas of tumors. They had also been modified to produce proteins useful in tumor treatment, such as the prodrug converting enzyme thymidine kinase by Pawelek, J., et al., Cancer Res. (1997) 57:4537-4544. The use of *Salmonella* in the treatment of tumors, however, was effectively prevented by the generation of sepsis due to the induction of tumor necrosis factor α stimulated by lipid A. Low, *et al.,* were able to disrupt the *msb* gene in *Salmonella* to reduce TNFα induction so as to override the sepsis inducing capacity of the bacterium, while retaining its antitumor activity. These authors showed that administration of the modified bacteria to mice with melanoma resulted in tumors that were <8% the size of tumors in untreated controls after 18 days.

Thus, it has been demonstrated in the art that anaerobic bacteria including facultative anaerobes can selectively home to the hypoxic areas of tumors and that modified forms of such bacteria lacking the toxic effects normally associated with them can safely be used in therapy.

The materials and methods for obtaining suitable expression of fluorescent proteins are readily available. Vectors containing various modified forms of GFP to provide various colors are marketed by Clontech. The Clontech vectors intended for mammalian cell expression place the GFP under control of the cytomegalovirus (CMV) promoter; such expression systems can also be used to label viral infectious agents. GFP expressing bacteria have been previously employed in a number of studies that were however not in intact, living animals (Wu, H., et al., Microbiol. (2000) 146:2481-2493; Ling, S.H.M., et al., Microbiol. (2000) 146:7-19; Badger, J.L., et al., Mol. Microbiol. (2000) 36(1):174-182; Kohler, R., et al., Mol. Gen. Genet. (2000) 262:1060-1069; Valdivia, R.H., et al., Gene (1996) 173:47-52; Valdivia, R.H., et al., Science (1997) 277:2007-2011; Scott, K.P., et al., FEMS Microbiol Ltrs. (2000) 182:23-27; Prachaiyo, P., et al., J. Food Protect. (2000) 63:427-433; Geoffroy, M-C., Applied & Env. Microbiol. (2000) 66:383-391). An example of such studies was the visualization of the *in vitro* infection of muscle tissue by the pathogenic *E. coli* Ol57H GFP (Prachaiyo, P., *et al., supra*). Another approach examined the mouse gastrointestinal tract after gavage infection by removal and fixation of the gastrointestinal tissue (Geoffroy, M-C., *supra*). Fish infected with GFP transduced *Edwardsiella tarda* were imaged for infection after removal of their organs (Ling, S.H.M., *et al., supra*). Genes associated with virulence and other infectious processes were evaluated by linkage to GFP expression (Ling, S.H.M., *et al., supra*; Badger, J.L., *et al., supra*; Kohler, R., *et al., supra*; Valdivia, R.H., *et al., supra* (1996).

### Disclosure of the Invention

The invention provides a means to monitor the targeting and proliferation of bacteria that can grow in hypoxic tumor areas and to evaluate the successful production of antitumor activities supplied by these bacteria. Simultaneously, the efficacy of the treatment can be evaluated by labeling and observing the tumor cells themselves. Thus, the invention provides a means to monitor and, if necessary, modify tumor treatment mediated by such bacteria.

Thus, in one aspect; the invention is directed to a method to verify the distribution of bacterial proliferation as confined to, and dispersed within, hypoxic tumor volumes which method comprises detecting, noninvasively, in a living subject the fluorescence emitted by a fluorescent protein contained within a bacterium administered to said subject.

In another aspect, the invention is directed to a method to monitor the production of an antitumor drug produced by an bacterium, said production being localized in the hypoxic volume of a tumor in a subject by detecting, noninvasively, in a living subject, the fluorescence of a protein fused to a therapeutic agent produced by a bacterium administered to the subject.

In a third aspect, the invention is directed to methods to monitor the effectiveness of tumor treatment using bacteria as a therapeutic agent and/or a delivery system for a therapeutic agent to the hypoxic volume of solid tumors which method comprises detecting the manner of proliferation or non-proliferation of tumors and metastases thereof by assessing the fluorescence emitted by tumors labeled with fluorescent proteins. This progress can be followed in conjunction with monitoring the therapeutic approaches as set forth above, by using various wavelengths of fluorescent emission.

### Modes of Carrying Out the Invention

The invention provides systems for monitoring the progress of infection by bacteria of hypoxic portions of solid tumors. The bacteria should be those which can survive in the anaerobic or essentially anaerobic hypoxic areas in these tumors. The bacteria must thus be either facultative or obligate anaerobes. Facultative anaerobes such as *E. coli* are preferred as they are less toxic to the subjects exposed to them than most obligate anaerobic bacteria. Advantage is taken of visible marker fluorescent proteins to label the bacteria so that their migration and colonization in solid tumors can be followed and so that localized production of therapeutic agents by these bacteria can be controlled and evaluated.

Since sufficient intensity can be achieved by the use of fluorescent proteins to observe the migration of fluorescent cells and production of protein in the intact animal, in addition to determining these aspects, the progress of tumor regression and metastasis or suppression thereof can be observed in the intact subject, since the tumor cells can themselves be labeled with a protein that fluoresces at a different wavelength.

The label used in the various aspects of the invention is a fluorescent protein, i.e., a protein that emits visible light when irradiated with an appropriate wavelength. The native gene encoding the seminal protein in this class, green fluorescent protein (GFP) has been cloned from the bioluminescent jellyfish *Aequorea victoria* (Morin, J., et al., J. Cell Physiol (1972) 77:313-318). The availability of the gene has made it possible to use GFP as a marker for gene expression. The original GFP itself is a 283 amino acid protein with a molecular weight of 27 kD. It requires no additional proteins from its native source nor does it require substrates or cofactors available only in its native source in order to fluoresce. (Prasher, D.C., et al., Gene (1992) 111:229-233; Yang, F., et al., Nature Biotechnol (1996) 14:1252-1256; Cody, C.W., et al., Biochemistry (1993) 32:1212-1218.) Mutation of the original GFP gene has been found useful to enhance expression and to modify excitation and fluorescence of the product, so that "GFP" in various colors, including reds, yellows and blues has been obtained. GFP-S65T (wherein serine at 65 is replaced with threonine) is particularly useful in the present invention method and has a single excitation peak at 490 nm. (Heim, R., et al., Nature (1995) 373:663-664); U.S. Patent No. 5,625,048. Other mutants have also been disclosed by Delagrade, S., et al., Biotechnology (1995) 13:151-154; Cormack, B., et al., Gene (1996) 173:33-38 and Cramer, A., et al., Nature Biotechnol (1996) 14:315-319. Additional mutants are also disclosed in U.S. Patent No. 5,625,048.

By suitable modification, the spectrum of light emitted by the GFP can be altered. Thus, although the term "GFP" is often used in the present application because of historical custom, the proteins included within this definition are not necessarily green in appearance, and should simply be referred to as fluorescent proteins. Various forms of "GFP" exhibit colors other than green and these, too, are included within the usage of "GFP" and are useful in the methods and materials of the invention. In addition, it is noted that green fluorescent proteins falling within the definition of "GFP" herein have been isolated from other organisms, such as the sea pansy, *Renilla reniformis.* Any suitable and convenient form of "GFP" of any color can be used to modify the infectious agents useful in the invention, both native and mutated forms.

In order to avoid confusion, the simple term "fluorescent protein" will often also be used; in general, this is understood to refer to the fluorescent proteins which are produced by various organisms, such as *Renilla* and *Aequorea* as well as modified forms of these native fluorescent proteins which may fluoresce in various visible colors. In general, the terms "fluorescent protein" and "GFP" are sometimes used interchangeably; however, sometimes specific other colors can be noted. The system is strictly mnemonic so that, for example, RFP refers to red fluorescent protein, YFP to yellow fluorescent protein, BFP to blue fluorescent protein, etc. A wide range of wavelength of visible light is emitted by these proteins depending on the specific modifications made.

Because fluorescent proteins are available in a variety of colors, imaging with respect to more than a single color can be done simultaneously. For example, two different bacterial agents or three different bacteria each expressing a characteristic fluorescence can be administered to the subject or a single bacterium could be labeled constitutively with a single color and a different color used to produce a fusion with a gene product. The nucleotide sequence encoding a fluorescent protein having a color different from that used to label the bacterium *per se* can be inserted at a genetic locus of a protein to be produced or as a fusion protein in a vector with a therapeutic protein to be produced.

The multiplicity of colors is particularly advantageous in the context of the invention. For example, the tumor itself may be labeled with a fluorescent protein of one color, the bacterium administered labeled with a structural or intracellular protein of a different color so that the location of the bacterium can be ascertained, and a protein product of the bacterium labeled with still a third color so that the level of production of this protein can be monitored. Thus, using whole body observation of a live animal, the location of the administered bacterium can be determined, the level of production of a therapeutic protein by that bacterium monitored, and the effect on the tumor monitored, all simultaneously.

The fluorescent proteins used in the present invention are of sufficient intensity that real time observation of the above phenomena in a living animal can be employed. This offers a major advance to the "blind" approach to bacterial delivery described in the prior art. Because the animal is alive, modifications to the treatment protocol to enhance its efficacy can advantageously be made when indicated by these observations.

If labeling of the tumor is desired, generation of the fluorescent protein in tumor cells has been described by the present applicants in U.S. patent 6,251,384 and 6,235,968. Briefly, viral vectors, preferably retroviral vectors, for expression of a fluorescent protein can be administered to subjects already harboring solid tumors. Alternatively, expression vectors may be injected intratumorally in the case of solid tumors. Model systems can be obtained by implantation into an immunocompromised or syngeneic animal of tumors which have been generated from cells modified to contain an expression system for a fluorescent protein. A variety of methods is described which result in labeling the tumor itself.

With respect to labeling the bacteria, the nucleotide sequence encoding the fluorescent protein may be introduced into the bacteria by direct modification, such as modification of the genome to locate the fluorescent protein encoding sequence in a suitable position under the control sequences endogenous to the bacteria, or may be introduced using appropriate expression vectors. The bacteria selected are bacteria that will survive and proliferate preferably selectively, if not completely specifically, in the hypoxic regions of solid tumors, leaving the remainder of the host animal substantially uninhabited preferably even if the bacteria are administered systemically. Preferably the bacterial culture will be dispersed in the hypoxic tumor volume as opposed to concentrated into small colonies.

The present invention provides a straightforward method to determine the most favorable bacterial hosts by direct observation *in situ.* Thus, the strain selected is labeled by insertion into the genome or by provision of an expression vector and administered to the animal. The pattern of proliferation in the tumor as opposed to other tissues can then be directly observed and the strain with the desired pattern chosen. A wide variety of candidates which are able to proliferate in hypoxic tumor volumes is known in the art, including *E. coli, Salmonella, Clostridium, Lactobacilli, Bifidobacteria* and the like. Suitable control sequences for expression in these systems are by now also well known in the art, or endogenous control sequences may be used.

In many cases, it may be desirable further to modify the bacteria to disable any ability to produce a toxic effect. This is more frequently the case for obligate anaerobes. If the bacteria secrete toxins, deletion or inactivation of the genes producing the toxin may be required; if the bacteria produce materials that engender undesired side effects, the genes encoding these materials may be inactivated or removed. The bacteria are modified either to express the fluorescent protein under control of a constitutive promoter as a constant feature of cell growth and reproduction, or the encoding sequence may be placed in the genome at particular desired locations, replacing endogenous sequences.

In addition to exerting its own inherent antitumor affects, the bacteria may also be modified to produce a therapeutic, such as IL2 or methioninase. In one embodiment the therapeutic protein is optionally generated as a fusion protein with a fluorescent protein. If the tumor and/or the bacteria are labeled, the color of the fluorescent protein in the fusion should be a different color than that chosen in either of the other two cases. Construction of fusions with fluorescent proteins are well known as markers, as described above. The expression system for the therapeutic protein, either alone or as a fusion with fluorescent protein, can be placed on a vector or in the genome of the bacteria and the control sequences may be constitutive or, in many cases, inducible and dependent on either *in situ* factors or externally supplied transcription factors.

One specific preferred example of a therapeutic protein is methioninase which exerts an antitumor affect when supplied intracellularly as disclosed in PCT publication WO 00/29589, or when supplied as a drug as described in U.S. patent 5,690,929, and in WO 94/11535. The recombinant production of methioninase is also disclosed in these documents.

In addition to its inherent effect on tumors, a therapeutic protein which is an enzyme can also be used, to release a toxic substance from a prodrug. For example, Miki, K., et al., Cancer Research (2001) 61:6805-6810 describe work which takes advantage of the toxicity of methyl selenol. This compound can be generated from selenomethionine by the action of methioninase. This article describes experiments in which the production of methyl selenol from selenomethionine by recombinantly generated methioninase kills cancer cells transformed with an expression system for this enzyme. The recombinant production of methioninase in the presence of selenomethionine can thus be used as a treatment for cancer.

In one embodiment of the invention which is provided for illustration only, bacteria such as *B. longum* or *C. novyi* are modified to disable production of any toxins. The detoxified bacteria are modified to contain an expression system for methioninase fused to a fluorescent protein. In addition, the bacteria are modified to contain an expression system for a fluorescent protein to label the bacteria *per se,* if desired. If the methioninase gene is constitutively expressed, this may be unnecessary as production of the methioninase itself will signal the presence of the bacteria. The thus modified bacteria are then administered to an experimental model subject harboring a tumor such as a tumor formed from human MDA-MB-435 breast cancer cells which have been, themselves, labeled with a fluorescent label of a color other than that used in the fusion protein. Alternatively, the tumor is indigenous and labeled using a viral expression vector as described in U.S. patent 6, 251,384 and 6,235,968 cited above.

If bacterial cells are used, the cells are injected to the breast cancer tumor directly; if spores are used, intravenous injection may also be used. Direct intratumoral injection of spores is also possible. The appropriately modified bacteria are administered to the subject in any practical manner. While in the case of an experimental tumor model, it may be necessary for the subject to be either immunocompromised or syngeneic with the tumor in order to provide the model, the administration of the bacteria *per se* does not require that the subject be immunocompromised. Thus, in the case of subjects bearing indigenous tumors, immunosuppression is unnecessary. Infection in the hypoxic tumor occurs readily in animals with intact immune systems. However, immunocompromised subjects may also be useful in studying the progress of the condition where the tumor is artificially introduced.

In one embodiment, the label for production ofmethioninase emits red fluorescence (RFP) that characteristic of the bacteria emits blue fluorescence (BFP) and that characteristic of the tumor emits green fluorescence (GFP).

In addition, if desired, selenomethionine is injected into the tumor, or systematically supplied. Production of methioninase *per se* and/or the presence of the bacteria *per se* are toxic to the tumor. The released methyl selenol is toxic not only to the immediate area in which the bacteria reside, but also diffuses more extensively to live tumor tissue. The progress of this therapy can be directly monitored by simultaneous imaging of RFP, GFP and BFP.

Fluorescent optical tumor imaging (FOTI) on whole body subjects externally permits real-time observation and monitoring of progression of infection on a continuous basis, in model systems or in subjects with indigenous tumors, and evaluation of the protocols. In subjects being treated, the availability of FOTI permits those devising treatment protocols to be informed on a continuous basis of the advisability of modifying or not modifying the protocol. Model systems are useful in the original design of treatment. In addition to external (FOTI) imaging, non-invasive endoscopic methods may also be used.

Suitable subjects for use as models are convenient laboratory animals such as rabbits, rats, mice.

### Preparation A

### Modification of Anaerobic Bacteria

A variant of the *A. victoria* green fluorescent protein was cloned into the BamHI and Notl sites of the pUC19 derivative pPD16.38 (Clontech, Palo Alto, CA) with GFP expressed from the *lac* promoter. The vector was termed pAV-GFP. pAV-GFP was transfected into *S. typhimurium* competent cells by standard methods, and transformed cells were selected by ampicillin resistance on agar plates. High expression *S. typhimurium*-GFP clones were selected by fluorescence microscopy.

### Example 1

*E. coli* were transfected with an expression vector for red fluorescent protein (RFP) and injected into nude mice which contained tumors labeled with green fluorescent protein (GFP). The animals were visualized by blue light excitation in a light box with a CCD camera and GFP-RFP filter; bacterial growth in the tumors was visualized by red and green light. The nude mouse tumor model wherein the tumor is labeled the GFP has been tested using prostate, melanoma, lung, colon, breast, renal, larynx, brain and pancreatic cancers.

### Example 2

*E. coli* were transfected with an expression system for a fusion protein consisting of methioninase coupled to GFP. The labeled bacteria were injected into tumors growing in nude mice which had been labeled with RFP. The mice were then administered selenomethionine by intratumoral injection. Two-color imaging as in Example 1 was used to visualize targeting of bacteria to the tumor as well as to follow the therapeutic effects.

### Example 3

GFP-labeled *Salmonella* were injected into an RFP-labeled U-87 human glioma in a nude mouse. A PBS solution (10 ul) containing 1x10⁸ GFP-labeled *Salmonella* was injected in the RFP-labeled U-87 human glioma. GFP-labeled *Salmonella* was imaged as in Example 1 in the RFP-labeled U-87 human glioma immediately after injection, and one day after injection. The GFP could be seen against the background of RFP at both times and had spread after one day.

### Example 4

GFP-labeled *Salmonella* was injected into RFP-labeled DU-145 human prostate tumors in nude mice. In one mouse, 1x10⁸ GFP-labeled *Salmonella* was injected in the RFP-labeled DU-145 human prostate tumor and imaged immediately after injection. In a second mouse, 2x10⁸ GFP-labeled *Salmonella* was injected in the RFP-labeled DU-145 human prostate tumor and imaged immediately after injection as in Example 1. In both cases the GFP could be seen against the background of RFP.

### Example 5

GFP-labeled *Salmonella* was injected into an RFP-labeled MDA MB-435 human breast tumor in a nude mouse. Buffer containing 2x10⁸ GFP-labeled *Salmonella* was injected into the RFP-labeled MDA MB-435 human breast tumor and imaged immediately after injection as in Example 1. The GFP could be seen against the background of RFP.

### Example 6

RFP-labeled *Salmonella* were able to grow in a GFP-labeled PC-3 human prostate tumor in a nude mouse. Buffer containing 3x10⁸ RFP-labeled *Salmonella* was injected into the GFP-labeled PC-3 human prostate tumor and imaged immediately after injection, and one day after injection as in Example 1. The GFP could be seen against the background of RFP in all images, and spreads over time.

### Example 7

In a second experiment, RFP-labeled *Salmonella* were able to grow in a GFP-labeled PC-3 human prostate tumor in a nude mouse. Buffer containing 2x10⁸ RFP-labeled *Salmonella* was injected in the GFP-labeled PC-3 human prostate tumor and imaged immediately after injection, one day after injection, and four days after injection as in Example 1. The GFP could be seen against the background of RFP in all images.

### Example 8

RFP-labeled *Salmonella* targeting and progressively growing in GFP-labeled PC-3 human prostate tumor growing in nude mice was also demonstrated by histology. RFP-labeled tissue was obtained containing *Salmonella* growing in the GFP-labeled PC-3 human prostate tumor four days after injection. The tumor tissue was fixed with 10% buffered formaline and processed for paraffin section and HE staining by standard methods. The RFP-labeled *Salmonella* could be seen progressively growing in the PC-3 tumor tissue and targeting the tumor cells.

### Example 9

In a second experiment, RFP-labeled *Salmonella* on PC-3 human prostate tumor growing in nude mice was demonstrated by histology. Sections were obtained as in Example 8. RFP-labeled *Salmonella* could be seen growing in the GFP-labeled PC-3 human prostate tumor four days after injection. In the untreated control, there was well-maintained tumor structure. After treatment with RFP-labeled *Salmonella,* the majority of tumor tissue was destroyed, and there was extensive necrosis in the tumor.

### Example 10

Buffer containing 10⁹ *E. coli* that express RFP was injected into PC-3 which was labeled with GFP and had been grown subcutaneously in nude mice for two weeks. Images were obtained as in Example 1. *E. coli*-RFP was visible in the PC-3-GFP tumor for at least 17 days.

## Claims

1. A method to evaluate the production of antitumor activities by anaerobic bacteria in a subject harboring a solid tumor, which method comprises:
observing the presence, absence or intensity of fluorescence in the solid tumor of said subject as a function of time, wherein said subject has been treated with anaerobic bacteria that express a first fluorescent protein of a first color; and
observing the presence or absence of regression or metastasis of the tumor in said subject, said tumor being labeled with a second fluorescent protein of a second color, different from the first color;
wherein the subject is a laboratory animal, which is a mouse, rat or rabbit, that has been modified to contain tumor cells that express said second fluorescent protein; and
wherein dispersement over time of fluorescence of said first fluorescent protein indicates the distribution of bacterial proliferation.

2. The method of claim 1 wherein said observing is by whole body fluorescent optical tumor imaging in the intact subject.

3. The method of claim 1 or 2 wherein said bacteria have been further modified to contain an expression system for a therapeutic protein.

4. The method of claim 3 wherein the therapeutic protein is produced as a fusion protein with a third fluorescent protein of a third color, different from the first and second colors.

5. A method to monitor the production of an antitumor drug which is a therapeutic protein produced by a bacterium in a subject, said production being localized in the hypoxic volume of a tumor, which method comprises:
monitoring over time the presence, absence or intensity of the fluorescence in a solid tumor of said subject that has been treated with anaerobic bacteria that have been modified to express a first fluorescent protein of a first color fused to a therapeutic protein;
whereby maintenance of the presence and intensity of the fluorescence of said first fluorescence protein in said tumor over time shows the therapeutic protein is present in the tumor;
and wherein the subject is a laboratory animal which is a mouse, rat or rabbit that has been modified to contain tumor cells that express a second fluorescent protein of a second color, different from the first color

6. The method of claim 5 wherein said monitoring is by whole body fluorescent optical tumor imaging in the intact subject.

7. The method of claim 5 or 6, which further comprises observing the regression or metastasis of the tumor.

8. The method of any of claims 3-7 wherein said therapeutic protein is methioninase.

9. A method to monitor the production of an antitumor drug which is a therapeutic protein produced by a bacterium in a subject, said production being localized in the hypoxic volume of a tumor, wherein the subject is a laboratory animal which is a mouse, rat or rabbit, which method comprises:
monitoring over time the presence, absence or intensity of the fluorescence in a solid tumor of said subject that has been treated with anaerobic bacteria that have been modified to express a first fluorescent protein of a first color, and a second fluorescent protein of a second color fused to said therapeutic protein;
whereby maintenance of the presence and intensity of the fluorescence of said second fluorescent protein in said tumor over time shows the therapeutic protein is present in the tumor.

10. The method of claim 9 wherein said monitoring is by whole body fluorescent optical tumor imagining in the intact subject.

11. The method of claim 9 or 10, which further comprises observing the regression or metastasis of the tumor, said tumor labeled with a third fluorescent protein of a third color, different from the first and second colors, wherein the subject is a mouse, rat or rabbit, that has been modified to contain tumor cells that express said third fluorescent protein.

12. The method of any of claims 9-11 wherein said therapeutic protein is methioninase.

## Patentansprüche

1. Verfahren zur Bewertung der Produktion von Antitumor-Aktivitäten durch anaerobe Bakterien in einem Individuum, das einen soliden Tumor trägt, wobei das Verfahren Folgendes umfasst:
Beobachten des Vorhandenseins, des Fehlens oder der Intensität der Fluoreszenz in dem soliden Tumor des Individuums als Funktion der Zeit, wobei das Individuum mit anaeroben Bakterien behandelt wurde, die ein erstes fluoreszierendes Protein einer ersten Farbe exprimieren; und
Beobachten des Vorhandenseins oder des Fehlens der Regression oder der Metastase des Tumors in dem Individuum, wobei der Tumor mit einem zweiten fluoreszierenden Protein einer zweiten Farbe markiert wird, die sich von der ersten Farbe unterscheidet;
wobei das Individuum ein Versuchstier ist, bei dem es sich um eine Maus, Ratte oder ein Kaninchen handelt, das so modifiziert wurde, dass es Tumorzellen enthält, die das zweite fluoreszierende Protein exprimieren; und
wobei die Streuung der Fluoreszenz des ersten fluoreszierenden Proteins über die Zeit die Verteilung der bakteriellen Proliferation anzeigt.

2. Verfahren nach Anspruch 1, wobei die Beobachtung durch optische Ganzkörper-Tumor-Fluoreszenzbildgebung im intakten Individuum erfolgt.

3. Verfahren nach Anspruch 1 oder 2, wobei die Bakterien weiter so modifiziert wurden, dass sie ein Expressionssystem für ein therapeutisches Protein enthalten.

4. Verfahren nach Anspruch 3, wobei das therapeutische Protein als Fusionsprotein mit einem dritten fluoreszierenden Protein einer dritten Farbe produziert wird, die sich von der ersten und zweiten Farbe unterscheidet.

5. Verfahren zum Überwachen der Produktion eines Antitumor-Medikamentes, bei dem es sich um ein therapeutisches Protein handelt, das von einem Bakterium in einem Individuum erzeugt wird, wobei die Produktion im hypoxischen Volumen eines Tumors lokalisiert ist, wobei das Verfahren Folgendes umfasst:
Überwachen des Vorhandenseins, des Fehlens oder der Intensität der Fluoreszenz über die Zeit in einem soliden Tumor des Individuums, das mit anaeroben Bakterien behandelt wurde, die so modifiziert wurden, dass sie ein erstes fluoreszierendes Protein einer ersten Farbe exprimieren, das an ein therapeutisches Protein gebunden ist;
wodurch die Aufrechterhaltung der Anwesenheit und Intensität der Fluoreszenz des ersten fluoreszierenden Proteins in dem Tumor über die Zeit zeigt, dass das therapeutische Protein in dem Tumor zugegen ist;
und wobei das Individuum ein Versuchstier ist, bei dem es sich um eine Maus, Ratte oder ein Kaninchen handelt, das so modifiziert wurde, dass es Tumorzellen enthält, die ein zweites fluoreszierendes Protein einer zweiten Farbe exprimieren, die sich von der ersten Farbe unterscheidet.

6. Verfahren nach Anspruch 5, wobei das Überwachen durch optische Ganzkörper-Tumor-Fluoreszenzbildgebung im intakten Individuum erfolgt.

7. Verfahren nach Anspruch 5 oder 6, das zudem die Beobachtung der Regression oder Metastase des Tumors umfasst.

8. Verfahren nach irgendeinem der Ansprüche 3 bis 7, wobei das therapeutische Protein Mehioninase ist.

9. Verfahren zum Überwachen der Produktion eines Antitumor-Medikamentes, bei dem es sich um ein therapeutisches Protein handelt, das durch ein Bakterium in einem Individuum produziert wird, wobei die Produktion im hypoxischen Volumen eines Tumors lokalisiert ist, und wobei das Individuum ein Versuchstier ist, bei dem es sich um eine Maus, Ratte oder ein Kaninchen handelt, wobei das Verfahren Folgendes umfasst:
Überwachen des Vorhandenseins, des Fehlens oder der Intensität der Fluoreszenz über die Zeit in einem soliden Tumor des Individuums, das mit anaeroben Bakterien behandelt wurde, die so modifiziert wurden, dass sie ein erstes fluoreszierendes Protein einer ersten Farbe und ein zweites fluoreszierendes Protein einer zweiten Farbe exprimieren, die an das therapeutische Protein gebunden sind;
wodurch die Aufrechterhaltung der Anwesenheit und Intensität der Fluoreszenz des zweiten fluoreszierenden Proteins in dem Tumor über die Zeit zeigt, dass das therapeutische Protein in dem Tumor zugegen ist.

10. Verfahren nach Anspruch 9, wobei das Überwachen durch optische Ganzkörper-Tumor-Fluoreszenzbildgebung im intakten Individuum erfolgt.

11. Verfahren nach Anspruch 9 oder 10, das zudem die Beobachtung der Regression oder Metastase des Tumors umfasst, wobei der Tumor mit einem dritten fluoreszierenden Protein einer dritten Farbe markiert ist, die sich von der ersten und zweiten Farbe unterscheidet, wobei es sich bei dem Individuum um eine Maus, Ratte oder ein Kaninchen handelt, das so modifiziert wurde, dass es Tumorzellen enthält, die das dritte fluoreszierende Protein exprimieren.

12. Verfahren nach irgendeinem der Ansprüche 9 bis 11, wobei das therapeutische Protein Methioninase ist.

## Revendications

1. Procédé pour évaluer la production d'activités anticancéreuses par des bactéries anaérobies dans un sujet porteur d'une tumeur solide, ledit procédé comprenant :
l'observation de la présence, l'absence ou l'intensité de fluorescence dans la tumeur solide dudit sujet en fonction du temps, où le sujet a été traité avec des bactéries anaérobies qui expriment une première protéine fluorescente d'une première couleur ; et
l'observation de la présence ou l'absence de régression ou de métastase de la tumeur dans ledit sujet, ladite tumeur étant marquée avec une deuxième protéine fluorescente d'une deuxième couleur, différente de la première couleur ;
où le sujet est un animal de laboratoire, qui est une souris, un rat ou un lapin, qui a été modifié de manière à contenir des cellules de tumeur qui expriment ladite deuxième protéine fluorescente ; et
où la dispersion au cours du temps de la fluorescence de ladite première protéine fluorescente indique la distribution de prolifération bactérienne.

2. Procédé de la revendication 1 dans lequel ladite observation est effectuée par imagerie de tumeur optique fluorescente du corps entier sur le sujet intact.

3. Procédé de la revendication 1 ou 2 dans lequel lesdites bactéries ont été en outre modifiées pour contenir un système d'expression pour une protéine thérapeutique.

4. Procédé de la revendication 3 dans lequel la protéine thérapeutique est produite sous forme de protéine de fusion avec une troisième protéine fluorescente d'une troisième couleur, différente des première et deuxième couleurs.

5. Procédé pour surveiller la production d'un médicament anticancéreux qui est une protéine thérapeutique produite par une bactérie chez un sujet, ladite production étant localisée dans le volume hypoxique d'une tumeur, ledit procédé comprenant :
la surveillance au cours du temps de la présence, l'absence ou l'intensité de la fluorescence dans une tumeur solide dudit sujet qui a été traité avec des bactéries anaérobies qui ont été modifiées pour exprimer une première protéine fluorescente d'une première couleur fusionnée avec une protéine thérapeutique ;
par le biais duquel le maintien de la présence et l'intensité de la fluorescence de ladite première protéine fluorescente dans ladite tumeur au cours du temps indique que la protéine thérapeutique est présente dans la tumeur ;
et où le sujet est un animal de laboratoire qui est une souris, un rat ou un lapin, qui a été modifié de manière à contenir des cellules de tumeur qui expriment une deuxième protéine fluorescente d'une deuxième couleur, différente de la première couleur.

6. Procédé de la revendication 5 dans lequel ladite surveillance est effectuée par imagerie de tumeur optique fluorescente du corps entier sur le sujet intact.

7. Procédé de la revendication 5 ou 6, qui comprend en outre l'observation de la régression ou la métastase de la tumeur.

8. Procédé de l'une quelconque des revendications 3 à 7 dans lequel ladite protéine thérapeutique est la méthioninase.

9. Procédé pour surveiller la production d'un médicament anticancéreux qui est une protéine thérapeutique produite par une bactérie chez un sujet, ladite production étant localisée dans le volume hypoxique d'une tumeur, où le sujet est un animal de laboratoire, qui est une souris, un rat ou un lapin, ledit procédé comprenant :
la surveillance au cours du temps de la présence, l'absence ou l'intensité de la fluorescence dans une tumeur solide dudit sujet qui a été traité avec des bactéries anaérobies qui ont été modifiées pour exprimer une première protéine fluorescente d'une première couleur, et un deuxième protéine fluorescente d'une deuxième couleur fusionnée avec ladite protéine thérapeutique ;
par le biais duquel le maintien de la présence et l'intensité de la fluorescence de ladite deuxième protéine fluorescente dans ladite tumeur au cours du temps indique que la protéine thérapeutique est présente dans la tumeur.

10. Procédé de la revendication 9 dans lequel ladite surveillance est effectuée par imagerie de tumeur optique fluorescente du corps entier sur le sujet intact.

11. Procédé de la revendication 9 ou 10, qui comprend en outre l'observation de la régression ou métastase de la tumeur, ladite tumeur étant marquée avec une troisième protéine fluorescente d'une troisième couleur, différente des première et deuxième couleurs, où le sujet est une souris un rat ou un lapin, qui a été modifié de manière à contenir des cellules de tumeur qui expriment ladite troisième protéine fluorescente.

12. Procédé de l'une quelconque des revendications 9 à 11 dans lequel ladite protéine thérapeutique est la méthioninase.
